# EUROPEAN PATENT SPECIFICATION

(11) **EP 1 441 788 B1**
(45) Date of publication and mention of the grant of the patent: **01.05.2019**
(21) Application number: 02769069.2
(22) Date of filing: 11.10.2002
(51) Int. Cl.: A61M 5/30

(54) **JET INJECTOR WITH HAND PIECE**
JET-INJEKTOR MIT HANDSTÜCK
INJECTEUR SANS AIGUILLE AVEC PIECE A MAIN

(30) Priority: 12.10.2001 US 329082 P; 01.03.2002 US 361198 P; 04.06.2002 US 386457 P
(43) Date of publication of application: 04.08.2004
(73) Proprietor: Pulse NeedleFree Systems, Inc., Lenexa, KS 66214 (US)
(72) Inventor: ROGATCHEV, Victor T., Voronezh, 394062 (RU); MCCALMON, Partick, Weaterhroby Lake, MO 64152 (US)
(74) Representative: Tomkins & Co
(86) International application number: PCT/US2002/032641
(87) International publication number: WO 2003/051433

(56) References cited:
- WO-A-92/10226
- DE-A1- 3 405 671
- GB-A- 1 132 065
- US-A- 2 695 611
- US-A- 3 859 996
- US-A- 3 933 155
- US-A- 4 342 310
- US-A- 5 062 830
- US-A- 5 954 689

## Description

### Field of the Invention

The present invention relates to needle-free injector systems, and more particularly to high work-load needle-free drug delivery devices for animal and human health applications.

### Background of the Invention

For many years, vaccination and administration of medicine has been accomplished by using syringes and needles. However, use of syringes and needles increases the risk of disease transmission among injection recipients. In addition, syringes and needles may cause tissue damage at the site of injection, thereby creating lesions and scar tissue. Particularly with the use of needle injection of animals, injection site lesions may result in losses of tens of millions of dollars each year to meat producers from reduced grade and carcass trim. Further, during injection needle tips may break causing residual needle fragments to remain in the subject. With animal use, this may further result in needle fragments entering into the food system. Disposable needles and syringes also create hazardous medical waste and waste disposal problems. A further drawback to disposable syringes and needles are the high costs when the units are provided for worldwide use. Many subjects, whether human or animal, have a strong aversion to needle injection. Accordingly, there exists a need for alternative methods of delivering medication to patients.

Alternative methods of delivering medication have been developed. One known method is to deliver medication using a needle-free injector. A needle-free injector delivers medication by providing a strong, high pressure blast of the medication through a small orifice, which causes a minute stream of the medication to exit the orifice at a high rate of speed, thereby allowing the medication to penetrate into the skin and subcutaneous tissues. A substantial amount of pressure is needed to create a high rate of speed of the medication. As a result, needle-free injectors are typically bulky and cumbersome to use. Further, accidental firing of the injector may cause misdosing of subjects and loss of medicine.

US Patent No. 3,859,996 discloses a multi-dose injector comprising a body portion, an ejector nozzle mounted at one end of said body portion, including a discharge orifice, an exit passage in said body portion leading to said orifice, powerized means for ejecting measured doses of liquid under pressure through the exit passage and out of the orifice against the epidermis of the recipient, where the apparatus has a pressure sensitive trigger mechanism whereby triggering of the dose releasing apparatus is effected only after a predetermined pressure of the trigger mechanism against the subject is sensed.

German Patent Publication No. DE3405671 discloses a device for setting up and maintaining the gas pressure in a vaccination gun operated with a high-pressure gas.

There is a need in the world health industry for a safe, economical, high work-load injection system to prevent and eradicate certain diseases in animals and humans.

### Summary of the Invention

The present invention as set out in the appended claims is directed to a jet injector system with a separate hand piece. It generally comprises a power unit, a medicine unit, an energy unit, a supply bottle, and a hand
piece. The hand piece and power unit are separate components connected to each other by a high pressure hose, which gives the user more flexibility. A valve assembly having a ball lock assembly and a needle assembly controls the release of medication from the high pressure hose into the hand piece and out a nozzle into the subject. Separating the components allows for a smaller hand piece that is easier to control and handle.

### Brief Description of the Drawings

Fig. 1 is a block diagram of major components of the injection system of the present invention.
Fig. 2 is a schematic view of one embodiment of the injection system of the present invention.
Fig. 3 is a schematic view of another embodiment of the injection system of the present invention.
Fig. 4 is a cross section of one embodiment of the amplifier of the present invention.
Fig. 5 is a cross section of another embodiment of the amplifier of the present invention.
Fig. 6 is a schematic view of the air distributor of the present invention.
Fig. 7 is a cross section of one embodiment of the medicine chamber of the present invention.
Fig. 8 is a cross section of another embodiment of the medicine chamber of the present invention.
Fig. 9 is a cross section of another embodiment of the medicine chamber of the present invention.
Fig. 10 is a perspective view of one embodiment of the hand piece of the present invention.
Fig. 11 is a side view of one embodiment of the core of the hand piece of the present invention.
Fig. 12 is a side view of one embodiment of the ball lock assembly of the core depicted in Fig. 11.
Fig. 13 is a side view of one embodiment of the needle assembly of the core of Fig. 11.
Fig. 14 was omitted from the application as filed.

### Detailed Description

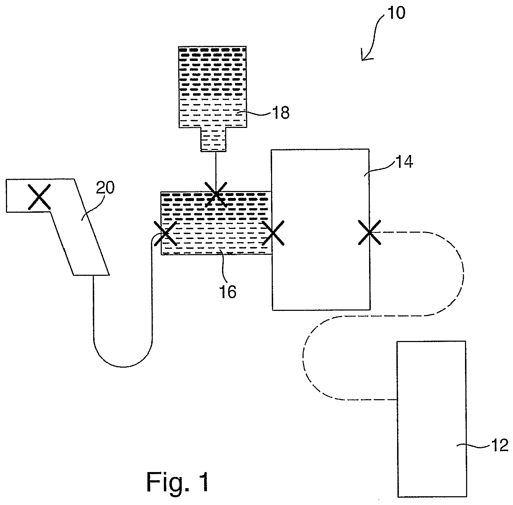

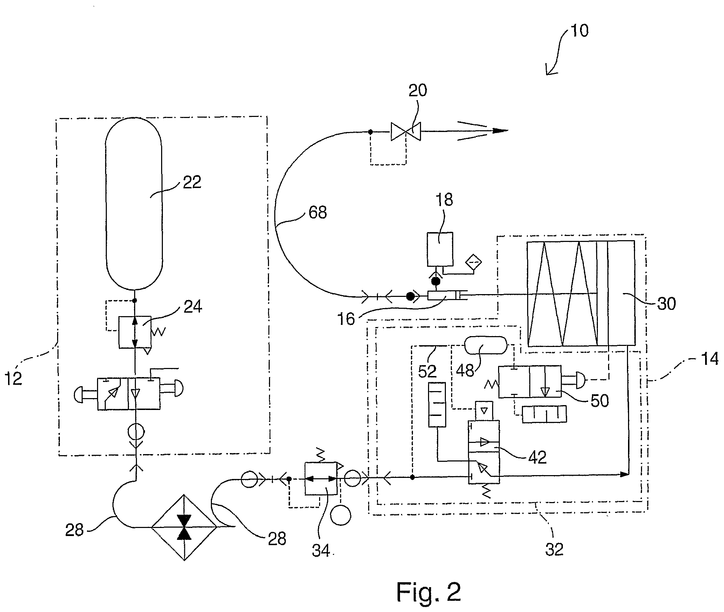

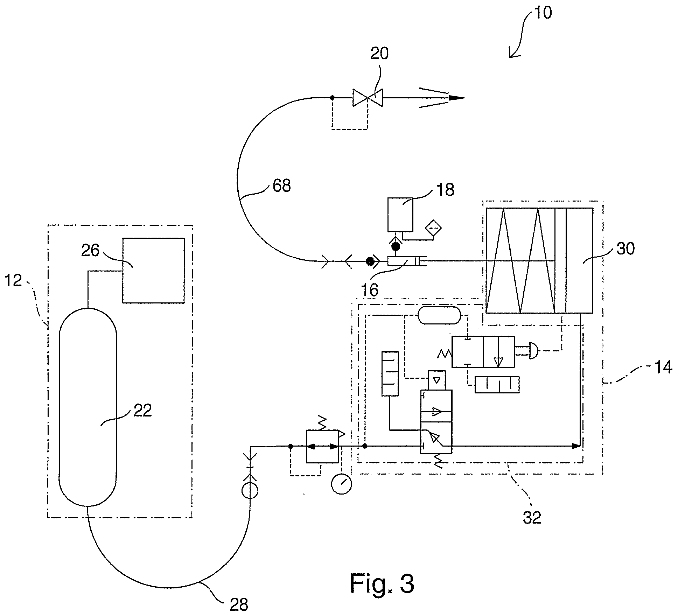

Fig. 1 depicts an injector 10 for injecting a human or an animal with a vaccine or other type of medication. The injector 10 has an energy unit 12, a power unit 14, a medicine unit 16, a supply bottle 18, and a hand piece 20. Critical interfaces, where each of these components connect, are illustrated as "X." A more detailed description of these interfaces is described below in reference to Figs. 2 and 3.

Fig. 2 depicts a schematic of the injector 10. The energy unit 12 may include a gas cylinder 22 connected to a regulator 24. In one embodiment, the gas cylinder 22 is a reusable carbon dioxide canister. In one embodiment, the regulator 24 regulates gas pressure from 50 to 120 psi. The energy unit 12 provides compressed regulated air or other gas to the power unit 14. This may be accomplished by a compressor 26 (Fig. 3) or by a pre-filled cylinder. The compressor 26 may be portable or stationary. If a portable compressor 26 is used then batteries or an AC power connection will supply electrical power (not shown). Gas cylinders 22 of various sizes may be utilized to hold varying amounts of gas. In one embodiment, the gas cylinder 22 holds liquid carbon dioxide.

The energy unit 12 supplies energy to the power unit 14. A hose 28 may be used to connect the energy unit 12 to the power unit 14. In one embodiment the hose length between the energy unit 12 and power unit 14 is approximately 300 mm.

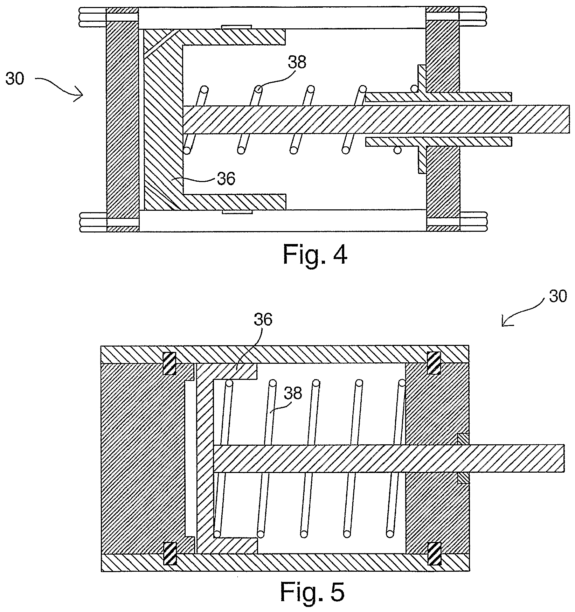

The power unit 14 comprises an amplifier 30 and an air distribution system 32. The amplifier 30 converts pneumatic energy to hydraulic pressure to pressurize the medicine unit 16 for both filling the medicine from the supply bottle 18 to the medicine unit 16 and dispensing the medicine from the medicine unit 16 to the hand piece 20. As depicted in Fig. 4, the amplifier 30 may include a power piston 36 connected to a refill spring 38. The pressure created by the amplifier 30 may be varied to accommodate varying subject sizes by using a regulator 34 (Fig. 2). Higher operational pressures will deposit the medication more deeply and quickly. Lower pressures will inject the dose less deeply. The amplifier 30 of Fig. 4 may be used as a build able amplifier. Fig. 5 depicts an alternate embodiment of the amplifier 30 of the present invention. The amplifier of Figure 5 may be a disposable amplifier 30 designed for a predetermined service life.

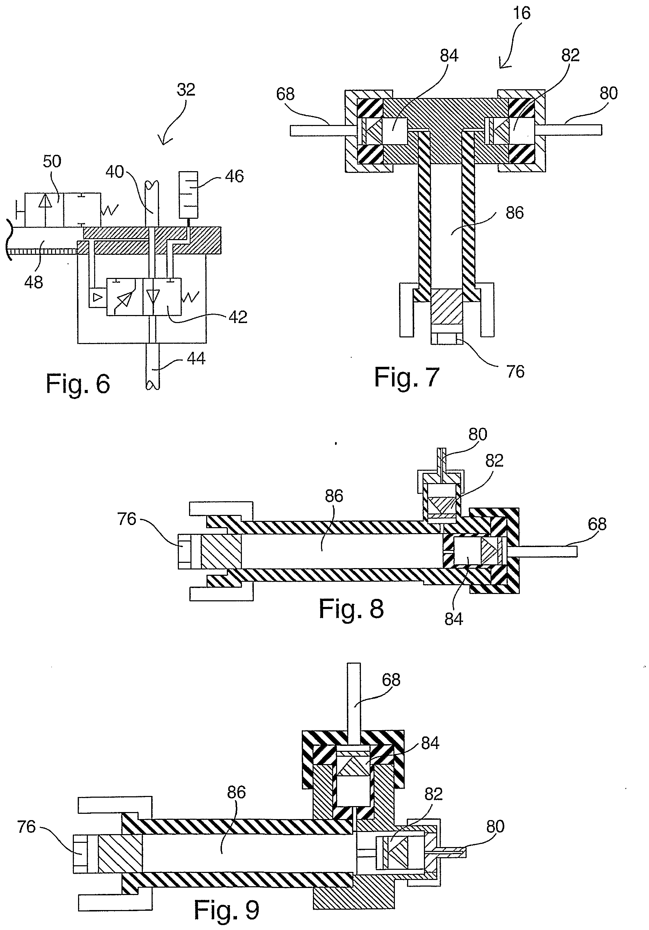

The air distribution system 32 supplies and controls the air flow into and out of the amplifier 30. As depicted in Fig. 6, gas travels through a supply line 40 from the regulator 34 (Fig. 2) into the air distribution system 32, first entering a three way piloted valve 42, which has two positions or a vent valve 50. In position one, the piloted valve 42 exhausts the air from the amplifier 30 to the atmosphere via a muffler 46. In position two, the piloted valve 42 releases air to an exit line 44 to the amplifier 30. When the system is not pressurized, compressed air travels from the supply line 40 into a pilot chamber 48 through a narrow passage 52 that controls the refill rate. The gas in the pilot chamber 48 is controlled by the vent valve 50. The vent valve 50 is mechanically actuated by the movement of the power piston 36 in the amplifier 30.

When the injector 10 is pressurized, vent valve 50 is closed and air from regulator 34 travels to piloted valve 42. The piloted valve 42 is in the second position when the injector 10 is pressurized and directs the air to the back side of power pistOn 36 in amplifier 30. When power piston 36 reaches the end of its stroke after an injection, no pressure remains in piloted valve 42, thereby causing piloted valve 42 to change to the first position allowing air from amplifier 30 to exhausts out of muffler 46. The vent valve 50 then closes and the three way piloted valve 42 opens back to the second position, causing air to travel to the amplifier 30.

The amplifier 30 then connects to the medicine unit 16 and may be integral with the medicine unit 16 as depicted in Fig. 1. The medicine unit 16 includes a piston rod 76 that controls the movement of medication within the medicine unit 16. The medicine unit 16 is attached to or connected to the supply bottle 18. The supply bottle 18 may be a conventional bulk medicine bottle 18 that is connected to the medicine unit 16 via an inlet tube 80 and a standard vent spike assembly (not shown). Alternatively, flexible medicine pouches (not shown) can be utilized in which only an un-vented spike assembly would be required. It is possible that the vent spike assembly be modified to provide proprietary connections for specific medications and to ensure that medications are not used with the wrong injector 10.

As depicted in Fig. 7, the medicine unit 16 includes an inlet valve 82 to receive the medicine from the inlet tube 80 and an outlet valve 84 to control the flow of the medicine out of the medicine unit 16 and into a high pressure hose 68 to the hand piece 20. A high pressure chamber 86 enclosed on one end by the piston rod 76 is located between the inlet valve 82 and the outlet valve 84 to store the medication after filling the medicine unit 16 and prior to injection. The size of the high pressure chamber 86 may be adjusted to accommodate various doses. In one embodiment, the diameter of high pressure chamber 86 is 8 mm and the diameter of the high pressure hose 68 is 2 mm. In one embodiment, the high pressure chamber 86 is sized to propel a 2 ml dose of medication to the subject.

In one embodiment, the piston rods 76 and 36 of the medicine unit 16 and the amplifier 30, respectively, move as one unit in both directions (fill and expel). For example, when pressure is applied to the piston 36 of the amplifier 30 from the air distribution unit 32, the piston 76 of the medicine unit 16 is also retracted within the high pressure chamber 86 causing the medication from the supply bottle 18 to be withdrawn into the high pressure chamber 86 and pressurized for injection. Upon activation of the injector 10, the piston rod 76 and the piston 36 are released forcing the pressurized medication within the high pressure chamber 86 through the outlet valve 84 into high-pressure hose 68 to the hand piece 20. In one embodiment, the high-pressure hose 68 has pressure capabilities of 10,000 psi or more. In yet another embodiment, the high-pressure hose 68 located between the medicine unit 16 and the hand piece 20 is 1300 mm +/-20mm in length. Figs. 8 and 9 depict alternate embodiments of the medicine unit 16 of the present invention, wherein the inlet valve 82 and the outlet valve 84 are located in different configurations.

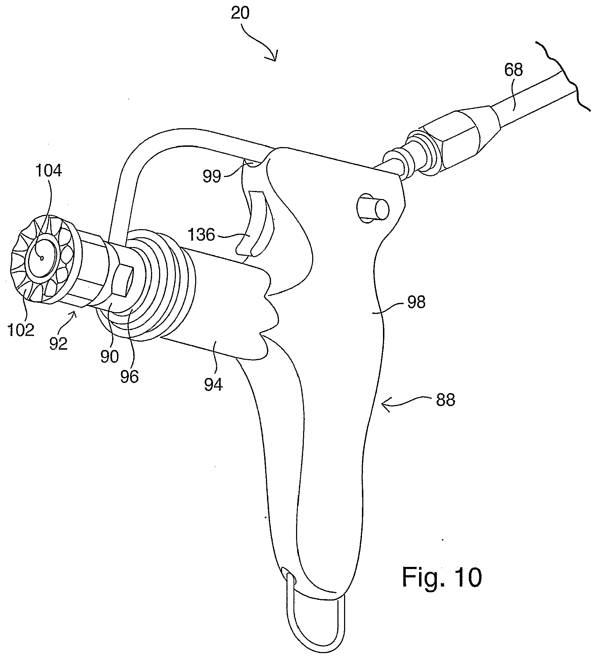

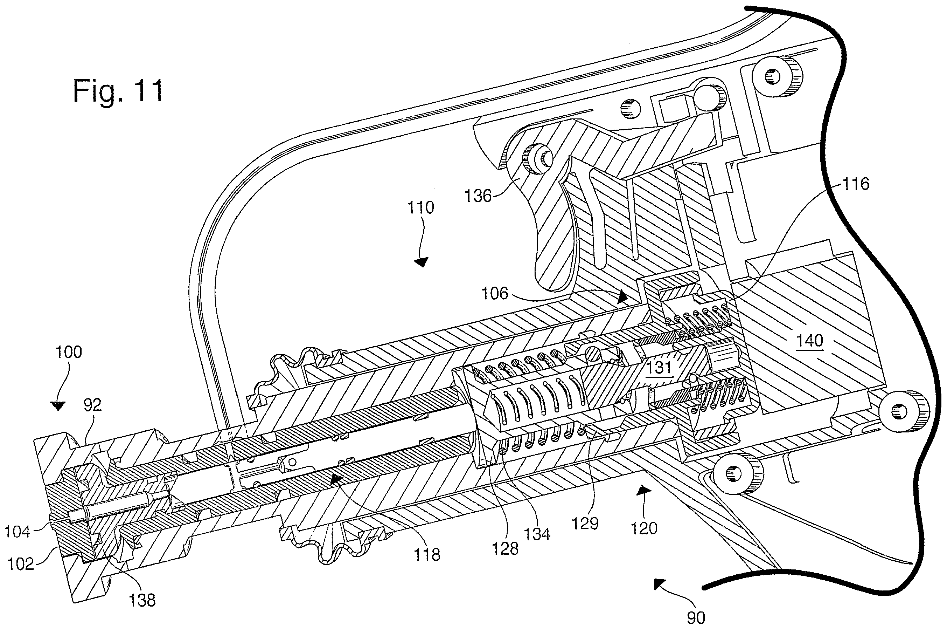

Fig. 10 depicts one embodiment of the hand piece 20 of the present invention. The hand piece 20 includes a body 88 and a core 90. The body 88 includes a housing 94 having an open end 96 and a closed end 98. The core 90, which rests at least partially within the housing 94 of the body 88, connects to the medicine unit 16 through the high pressure hose 68. In one embodiment, the hose 68 is made of stainless steel. The body 88 of the hand piece 20 may include a channel 99 which positions the hose 68 on the body 88 of the hand piece 20 to prevent
rotation of the core 90 within the body 88. The core 90 has a distal end 100 having a nozzle 102 held in place by a nozzle nut 92 and a proximal end 106 (Fig. 11). The entire core 90 may slide in the housing 94 so that the injector 10 will be actuated only when the nozzle 102 comes in contact with the subject being injected. In one embodiment, a bellows seal (not shown) may be utilized to prevent foreign material from entering the housing 94.

The nozzle 102 may be located distal to the nozzle nut 92 and has an orifice 104 that releases the medication into the subject. The velocity of medication to be injected may be controlled by varying the diameter of orifice 104 or by varying the pressure in the high pressure chamber 86. In one embodiment, the orifice 104 has a diameter of 0.2-0.36 mm. In another embodiment, the nozzle 102 includes a ruby orifice 104.

The front face of the nozzle 102 may have a textured surface. For example, in one embodiment depicted in Fig. 10, the nozzle 102 has a scalloped surface. The scalloped nozzle may be designed to ensure that there is no relative movement between the nozzle 102 and the subject. This is particularly important when attempting to vaccinate or give injections to moving subjects. In one embodiment, a protective cap (not shown) covers the nozzle 102 to prevent debris or blood from the subject from entering through the orifice 104 into the hand piece 20.

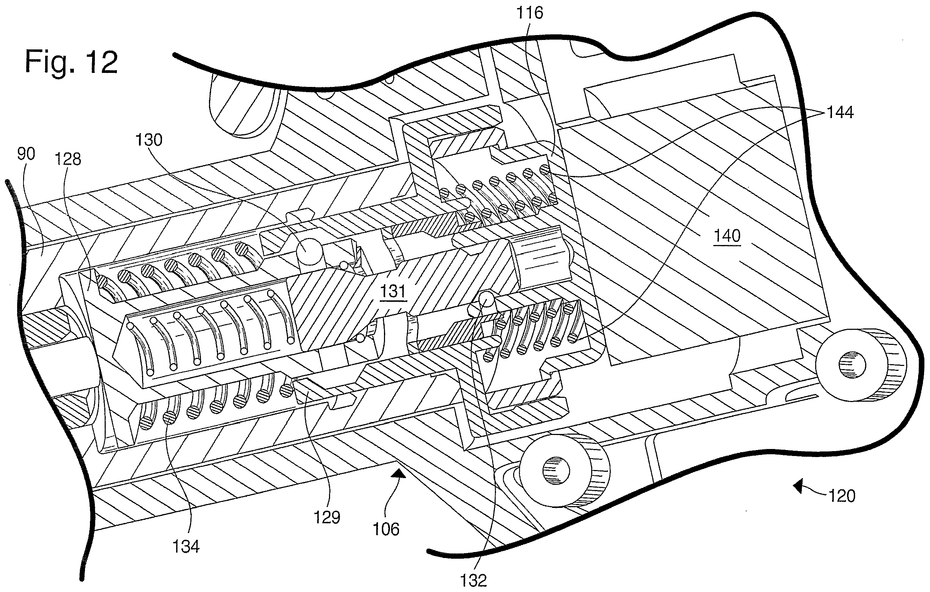

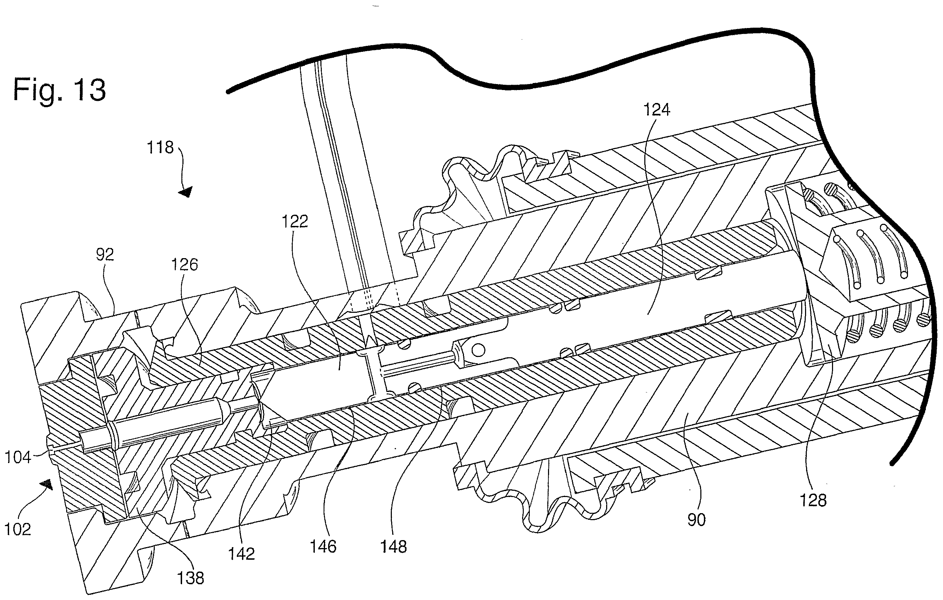

As depicted in Figs. 11 through 13, the core 90 further includes a valve assembly 110 located within the core 90 to control the release of medicine from the medicine unit 16 to the hand piece 20 and out to the subject. The valve assembly 110 includes a front seal plug 138, a needle assembly 118 (Fig. 13), a ball lock assembly 120 (Fig. 12), and a rear housing plug 140. As depicted in Fig. 12, the ball lock assembly 120 includes a button 116 at the proximal end 106 of the core 90, a front separator 128, a first ball lock 130, a central core pin 131, a second ball lock 132, a main spring 134, and a plurality of biasing springs 144. The ball lock assembly 120 attaches to the core 90 via a ball lock frame 129. The main spring 134 is located between the front separator 128 and the ball lock frame 129.

The needle assembly 118 is located between the nozzle nut 92 and the front separator 128 (Fig. 11). Lateral movement of the needle assembly 118 within the core 90 controls the release of medicine from the hand piece 20. The needle assembly 118, depicted in further detail in Fig. 13, includes a front portion 122 and a back portion 124 housed in an insert sleeve 126 of the core 90. The back portion 124 rests against the front separator 128. The front portion rests against a saddle 142 of the front seal plug 138 when the injector 10 is not activated. The needle assembly
118 may further include pressurizing chambers 146 and 148 that must be pressurized in order to open the needle assembly 118. The front seal plug 138 provides an exit for the medication from the high-pressure hose 68 to the orifice 104 when the needle assembly 118 opens.

The needle assembly 118 is designed so that it senses pressure within the high pressure chamber 86. If the high pressure chamber 86 is not adequately pressurized (i.e. when the high pressure chamber 86 is not fully dosed), then the pressurizing chambers 146 and 148 are not pressurized and the needle assembly 118 blocks passage of the medicine from the high pressure hose 68 to the core 90 and will not open to release the medicine. When the needle assembly 118 senses an adequate level of pressure within the high pressure chamber 86, the needle assembly 118 provides a passage for the medication from the high pressure hose 68 through the hand piece 20 and out the nozzle 102.

When pressurizing chambers 146 and 148 are pressurized by the hydraulic pressure supplied from the high-pressure hose 68, movement of ball lock assembly 120 controls the opening and closing of the needle assembly 118. When the nozzle 102 is pressed against a subject, the core 90 moves toward the closed end 98 of the housing 94 causing the button 116 of the ball lock assembly 120 to contact a rear housing plug 140, thereby causing the button 116 to depress. The button 116 contacts the rear housing plug 140 by a push or pull button core concept. In the embodiment depicted in Figs. 11-13, the core 90 pushes the button 116 into the rear housing plug 140. Depression of the button 116 against the rear housing plug 140 causes the central core pin 131 to move forward toward the distal end 100, allowing the front ball lock 130 to release into the ball lock frame 129. Release of the front ball lock 130 allows the front separator 128 to move toward the proximal end 106 of the core 90 due to the hydraulic pressure pushing on the back portion 124 of the needle assembly 118. When the pin 131 reaches the end of the stroke toward the distal end 100, biasing springs 144 force the central pin 131 to move back toward the proximal end 16, thereby releasing second ball lock 132 and allowing the button 116, front separator 128, and central pin to move independently from each. Movement of the back portion 124 toward the proximal end 106 of the core 90 causes the front portion 122 of the needle assembly 118 to move away from the saddle 142 toward the proximal end 106. When the front portion 122 moves away from the saddle 142, the medication can flow through the seal plug 138 and exit the orifice 104.

As long as the central pin 131, front separator 128, and the button 116 move independently, the injector 10 cannot be actuated again. The flow of medication continues to exit the needle valve 118 until pressure in the pressurizing chambers 146 and 148 drops below a critical level (approximately 2000 psi). When the pressure reaches this level, the main spring 134 pushes the front separator 128 into the back portion 124 of the needle assembly 118 toward the distal end until the front portion 122 reaches the seal plug 138, thereby closing the path for to the orifice 104. Once the front portion 122 of the needle assembly 118 reseats against the saddle 142, the first ball lock 130 reengages to its initial position. When the hand piece 20 is released from the patient being injected, the biasing springs 144 in the ball lock assembly 120 are released and the second ball lock 132 returns to the locked position. The first ball lock 130 cannot reopen until the button 116 is released again.

The ball lock assembly 120, as disclosed above, operates by pushing the button 116 with the sliding core 90. Alternately, the ball lock assembly 118 may be designed to operate by pulling a pin (pull core). In the pull core, the entire core 90 does not move. Instead, the core 90 may include a sliding ring at the front of the injector 10 connected to a pin extending from the core assembly with bails or push rods (not shown).

The injector 10 may be designed to operate only if the hand piece 20 is held properly. The injector 10 may be designed so that in order to give the injection to a subject, the hand piece 20 must be pushed against the subject. The correct amount of force may cause the injector 10 to fire the medication into the subject. For example, as depicted in Figs. 11 through 13, the sliding core 90 serves as at least one level of safety to prevent injection of the medication when the hand piece 20 is not held against a subject. In another embodiment, at least one other level of safety is included wherein the injector 10 may require the operator to hold a safety 136 (Fig. 10) down to allow the injector 10 to work, so that if the user drops the hand piece 20, the injector 10 will not fire. For example, as depicted in Fig. 11, a safety 136 extends from the exterior of the housing 94 of the body 88 into the interior of the housing 94 to impede movement of the button 116 when the injector 10 is not in use. When the operator is ready to use the injector 10, the operator releases the safety 136 to permit sliding movement of the button 116. In yet another embodiment, an ON/OFF safety may be included on the hand piece 20.

The hand piece 20 may take any shape suitable for injection into a subject. The design of the hand piece 20 is not intended to be limited to the embodiments depicted in the figures. In one embodiment, the hand piece 20 has an ergonomic design to minimize repetitive hand motion and fatigue and adapted to left- or right-handed operation. An ergonomic hand piece 20 is designed to fit in the hand of the operator for ease of use. In one embodiment, the hand piece 20 may include a counter (not shown) to count each injection cycle. The counter may be resettable or non-resettable.

In one embodiment, not shown because Fig. 14 was omitted from the application as filed, the injector 10 is assembled and stored in an adjustable vest, adaptable for both right- and left-handed users. The vest may be designed to assure comfortable fit and weight distribution for users of all sizes and statures. The vest may include two chest straps attached to a back portion and a waist portion. Belts may further secure the chest straps and the waist portion to the operator. The vest may further include pockets to accommodate the amplifier 30, the gas cylinder 22, supply bottles 18 of any size, markers, gloves, and other materials. A skilled artisan would recognize that any number of pockets could be utilized with the present invention to accommodate the individual components. Further, these pockets may be placed at any location on the vest. In one embodiment, one pocket is located on each chest strap to hold the supply bottles 18 and two pockets are located on the waist portion to hold the amplifier 30 and the gas cylinder 22. The vest may also include a clip that holds the hand piece 20. Any configuration of the vest is foreseen including but not limited to a one-piece vest, resembling a clothing article that may be fastened by any type of fastener (button, zipper, ties, etc.) or a vest as described above but with only one chest strap.

In operation, the injector 10 of the present invention is capable of performing multiple actuations. First, the operator attaches a supply bottle 18 to the medicine unit 16. Once the supply bottle 18 is connected, the power unit 14 automatically primes to the operational system pressure. The pilot valve 42 of the air distribution system 32 supplies pressure to the power piston 36 in the amplifier 30, thereby pressurizing the high pressure chamber 86 of the medicine unit 16. Once the high pressure chamber 86 is pressurized above 500 psi, the operator can approach the subject. The operator places the hand piece 20 in his hand to release the safety 136 and presses the hand piece 20 against the subject with a predetermined minimum force. In one embodiment, the predetermined minimum force is 2 psi. The predetermined minimum force, however, may be adjustable. When the pressure in the high pressure chamber 86 is at operational pressure and the nozzle 102 is pressed into the subject by the operator, core 90
applies pressure to the button 116 and causes the central pin 131 to move and disengage the first ball lock 130. The force holding the front portion 122 of the needle assembly 118 against the saddle 142 is relieved and the needle assembly 118 opens. When the needle assembly 118 opens, the medicine in the high-pressure chamber 86 is able to flow through the outlet valve 84 of the medicine unit 16, through the high pressure hose 68, through the needle assembly 118 and out the orifice 104.

As the fluid in the high pressure chamber 86 escapes, the power piston 36 is driven by the air distribution system 32 which maintains the pressure in the high pressure chamber 86 during injection. The power piston 36 continues to move forward causing a predetermined amount of medication to be dispensed until the pressure in the high pressure chamber 86 drops below minimum pressure. This occurs as the power piston 36 reaches the end of the stroke and starts to retract being pushed back by the power spring 38 in the amplifier 30. The valve assembly 110 will remain open until the pressure in the high pressure chamber 86 drops below the minimum pressure, typically 500 psi. In one embodiment, the needle assembly 118 remains open for approximately 100-200 milliseconds. Once the pressure in the high pressure chamber 86 and pressurizing chambers 146 and 148 drops below minimum, the force of main spring 134 against the needle assembly 118 overcomes the minimum pressure in chambers 86, 146, and 148 and closes regardless of whether the nozzle 102 is pressed against a subject. The hand piece 20 is then removed from the skin of the subject and the valve assembly 110 resets. As the power piston 36 returns to its initial position for the next injection, the predetermined amount of medication is drawn from the supply bottle 18 to the medicine unit 16, the high pressure chamber 86 is repressurized, and the injector 10 is ready for the next injection. If the pressure in the high pressure chamber 86 is below minimum pressure, pressing the nozzle nut 92 against the subject will not cause the valve assembly 110 to open because the force of the main spring 134 continues to overcome the minimum pressure.

The following example illustrates the methods and devices of the present inventions, which should not be construed as limiting in any way.

### EXAMPLE 1

The objective of the following study was to compare the serological responses induced by vaccination, the tissue reaction and general health-related safety between traditional injection by hypodermic needle and a needle-free injection device.

### Materials and Methods

Two swine weaning groups were studied and designated Trial 1 and Trial 2. Pigs were bled, tagged, tattooed and randomly assigned to treatment groups (needle-free, needle, none/control) at 4-5 weeks of age. For the hypodermic needle injections, an 18 gauge x 5/8 inch (first vaccination) or 1 inch (second and third vaccinations) needle was used to ensure intramuscular deposition of the vaccines. Needles were changed at least every 6 pigs. The injector described below in Table 1 was utilized for the needle-free injections.

The pigs were vaccinated with two doses of commercial *Mycoplasma hyopneumoniae* vaccine (RespiSure®, Pfizer Animal Health) at 5-6 weeks of age and again 2 weeks later, and with a commercial pseudorabies virus vaccine (PrVac+®, Pfizer Animal Health) at 9-10 weeks of age. Blood samples were collected at 11-13 days after the second mycoplasma vaccination and 23-25 days after the PRV vaccination.

For safety evaluation, the pigs were weighed periodically, and injection sites were observed and palpated 2 days after each vaccination and at each bleeding. In addition, injection sites were thoroughly dissected at slaughter. Data was subjected to analysis of variance to determine statistical significance.

**Table 1**

| Characteristics | Requirement |
|---|---|
| | |

| Energy source | Pneumatic (Compressor) |
|---|---|
| Storage tank pressure range | 0.75 to 1.0 MPa (109 to 145 psi) |
| Regulated Pressure range | 0.35 to 0.70 MPa (50 to 100 psi) +/- 5% |
| Regulator repeatability | +/-2% |
| Steady state flow rate | 7 Unnin (1.9g/min) @ 0.70MPa |
| | |

| Jet energy | User Adjustable |
|---|---|
| Maximum Fluid Pressure | 70 MPa (10,000 psi) |
| Cut-off Fluid Pressure | 14 MPa (2000 psi) |
| Orifice Diameter | Changeable |
| Diameter options | 0.16-0.36 mm |
| | |
| Injection Type | Subcutaneous & Intra Muscular |
| | (Adjusted by orifice and pressure) |
| | |

| Injectable Medication supply type | Remote mounted Bottles |
|---|---|
| Bottle Sizes | 250, 500 & 1000 ml |

| Dosage | Changeable discrete settings (available) |
|---|---|
| Dose settings | 1.0, 1.5, 2.0, and 2.5 ml |
| Method of changing settings | Field interchangeable components (inserts) |
| Injection Rate | 1200 injections/hour |
| Burst rate | 4 injections / 6 sec |
| | |
| Counter device to count each injection cycle | Required - six digit non-resettable |
| | |
| System Weight (w/o Injectable medication) | 7.7 kg (17 lbs) - Goal |
| | |

| Hog characteristics | |
|---|---|
| Age | Weaned to Breeding Stock |
| Variety | Standard market hogs |

### Results

Serological data is presented in Table 2. All pigs were seronegative for *M hyo.* and PRV prior to vaccination. The serological responses of vaccinated pigs, regardless of injection type, were significantly greater than the control pigs (P<0.05). There was no difference between the two injection types with regard to the serological responses induced by either vaccine.

Evaluation of the injection sites at slaughter indicated no injection site lesions in pigs from any of the three treatment groups. There was no difference in weight gain between the three treatments.

**Table 2**

| Injection | | *M. hyo* | *OD values* | *PRV* |
|---|---|---|---|---|
| Trial | Type | Test 1 | Test 2 | S/P Ratio |
| 1 | Needle-free injector | 0.559 | 0.407 | 1.259 |
| | Needle | 0.515 | 0.426 | 1.124 |
| | Control | 0.038 | 0.073 | 0.016 |
| 2 | Needle-free injector | 0.449 | 0.241 | 1.874 |
| | Needle | 0.377 | 0.259 | 2.116 |
| | Control | 0.075 | 0.047 | 0.037 |

The pigs injected with the needle-free injector exhibited serological responses equivalent to those achieved with a needle injection. Further, injection with the needle-free injector did not result in more tissue damage when compared to conventional needle injection. Similar responses have been observed in trials with combination vaccines containing inactivated *M hyo.* and other viral and bacterial antigens.

Although the present invention is described by reference to a single and exemplary embodiment, and the best mode contemplated for carrying out the present invention has been shown and described, it is to be understood that modifications or variations in the structure and arrangements of this embodiment other than those specifically set forth may be achieved by those skilled in the art and that such modifications are to be considered as being within the overall scope of the present invention, which is defined by the claims.

## Claims

1. A jet injector (10) with a separate hand piece (20) for administering a medicine to a subject, comprising:
the separate hand piece (20) having a body (88) and a core (90) at least partially within the body (88), wherein said core (90) has a nozzle (102) on a distal end, wherein the nozzle (102) has an orifice (104) that releases medicine from the core (90) into the subject;
a medicine unit (16) having an inlet valve 82 configured to receive medicine, an outlet valve configured to control the flow of medicine out of medicine unit (16) and a high pressure chamber (86) in fluid communication with the core (90) of the separate hand piece (20) via a high pressure hose (68) to supply medicine at a high velocity to the core (90) of the hand piece (20), the medicine unit (16) further having a piston (76) located at one end of the high pressure chamber (86);
and wherein the hand piece (20) further comprises a valve assembly (110) located in the core (90) for controlling the release of medicine from the medicine unit (16) to the hand piece (20), wherein the valve assembly (110) comprises a ball lock assembly (120) and a needle assembly (118),
wherein the ball lock assembly (120) comprises a button (116), wherein activation of the button (116) causes the ball lock assembly (120) to release, wherein when the core (90) is pressed against the subject, the core (90) contacts the button (116) and causes the ball lock assembly (120) to release,
wherein release of the ball lock assembly (120) provides for movement of the needle assembly (118), wherein the movement of the needle assembly (118) controls the release of the medicine from the medicine unit (16) to the hand piece (20) and out of the core (90) of the hand piece (20),
a supply bottle (18) in communication with the medicine unit (16), wherein the supply bottle (18) supplies the medicine to the medicine unit (16);
a power unit (14) connected to said medicine unit (16) for creating high pressure in the high pressure chamber (86), the power unit (14) having an amplifier (30) and an air distribution system (32),
wherein the amplifier (30) converts pneumatic energy to hydraulic pressure to pressurize the high pressure chamber (86) of the medicine unit (16) and wherein the amplifier (30) comprises a piston (36),
wherein the air distribution system supplies and controls the airflow into amplifier (30) via a valve (42) to provide pressure against piston (36) of amplifier (30) necessary for amplifier (30) to pressurize the high pressure chamber (86) of the medicine unit (16),
wherein the piston (76) of the medicine unit (16) and piston (36) of amplifier (30) are configured to move as one unit, such that when pressure is applied to piston (36) of amplifier (30) from air distribution system (32), the piston (76) of medicine unit (16) is retracted within high pressure chamber (86), causing the medicine from supply bottle (18) to be withdrawn into high pressure chamber (86) and pressurized for injection and such that upon opening of needle assembly (118), piston (76) of the medicine unit (16) and piston (36) of amplifier (30) are released and force the pressurized medicine within the high pressure chamber (86) into high-pressure hose (68) to the hand piece (20); and
an energy unit (12) having a gas cylinder (22), wherein the energy unit (12) supplies gas to the air distribution system (32) of the power unit (14).

2. The injector (10) of claim 1, wherein the ball lock assembly (120) further comprises a first ball lock (130), a main spring (134), and a second ball lock (132), wherein the first ball lock (130) controls the release of the main spring (134) and the second ball lock (132) controls reactivation of the injector (10).

3. The injector (10) of any one of claims 1 or 2, wherein the hand piece (20) further comprises a safety (136) in communication with the button (116), wherein when the safety (136) is engaged, the button (116) cannot release the ball lock assembly (120).

4. The injector (10) of any one of claims 1 to 3, wherein when the core (90) is pressed against the subject, the core (90) contacts the button (116).

5. The injector (10) of claim 4, wherein the core (90) is configured to push the button (116) to open the valve assembly (110).

6. The injector (10) of claim 4, wherein the core (90) is configured to pull the button (116) to open the valve assembly (110).

7. A kit for transdermally delivering a medicine to a subject, comprising:
the jet injector (10) of any one of claims 1 to 6; and
a vest (150) having at least one pocket (160) configured to hold at least one of the hand piece (20), a supply bottle (18), the power unit (14), or the energy unit (12).

8. The kit of Claim 7, wherein the vest (150) comprises at least three pockets (160) to hold each of the supply bottle (18), the power unit (14), and the energy unit (12).

9. The kit of claim 7 or claim 8, wherein the vest (150) further comprises at least one chest strap (152) connected to at least one back portion (154) and to at least one waist portion (156).

10. The kit of any of claims 7 to 9, wherein the vest (150) further comprises a clip (162) configured to hold the hand piece.

## Patentansprüche

1. Strahlinjektionsvorrichtung bzw. Impfpistole (10) mit einem separaten Handstück (20) zum Verabreichen einer Medizin an ein Lebewesen, welche Folgendes aufweist:
das separate Handstück (20) mit einem Körper (88) und einem Kern (90) zumindest teilweise innerhalb des Körpers (88), wobei der Kern (90) eine Düse (102) an einem distalen bzw. äußeren Ende hat, wobei die Düse (102) eine Zumessöffnung (104) hat, welche Medizin aus dem Kern (90) in das Lebewesen freigibt;
eine Medizineinheit (16) mit einem Einlassventil (82), das konfiguriert ist, um Medizin aufzunehmen, mit einem Auslassventil, das konfiguriert ist, um den Fluss der Medizin aus der Medizineinheit (16) zu steuern, und mit einer Hochdruckkammer (86) in Strömungsmittelverbindung mit dem Kern (90) des separaten Handstücks (20) über einen Hochdruckschlauch (68), um Medizin mit hoher Geschwindigkeit zum Kern (90) des Handstücks (20) zu liefern, wobei die Medizineinheit (16) weiter einen Kolben (76) hat, der an einem Ende der Hochdruckkammer (86) angeordnet ist;
und wobei das Handstück (20) weiter eine Ventilanordnung (110) aufweist, die in dem Kern (90) angeordnet ist, um das Abgeben von Medizin aus der Medizineinheit (16) an das Handstück (20) zu steuern, wobei die Ventilanordnung (110) eine Kugelverschlussanordnung (120) und eine Nadelanordnung (118) aufweist,
wobei die Kugelverschlussanordnung (120) einen Knopf (116) aufweist, wobei eine Aktivierung des Knopfes (116) bewirkt, dass die Kugelverschlussanordnung (120) freigibt, wobei, wenn der Kern (90) gegen das Lebewesen gedrückt wird, der Kern (90) in Kontakt mit dem Knopf (116) kommt und bewirkt, dass die Kugelverschlussanordnung (120) freigibt,
wobei das Freigeben der Kugelverschlussanordnung (120) für eine Bewegung der Nadelanordnung (118) sorgt, wobei die Bewegung der Nadelanordnung (118) das Ausgeben der Medizin aus der Medizineinheit (16) an das Handstück (20) und aus dem Kern (90) des Handstücks (20) steuert,
eine Vorratsflasche (18) in Verbindung mit der Medizineinheit (16), wobei die Vorratsflasche (18) die Medizin an die Medizineinheit (16) liefert,
eine Antriebseinheit (14), die mit der Medizineinheit (16) verbunden ist, um hohen Druck in der Hochdruckkammer (86) zu erzeugen, wobei die Antriebseinheit (14) einen Verstärker (30) und ein Luftverteilungssystem (32) hat,
wobei der Verstärker (30) pneumatische Energie in hydraulischen Druck umwandelt, um die Hochdruckkammer (86) der Medizineinheit (16) unter Druck zu setzen, und wobei der Verstärker (30) einen Kolben (36) aufweist,
wobei das Luftverteilungssystem den Luftfluss in den Verstärker (30) über ein Ventil (42) liefert und steuert, um einen Druck gegen den Kolben (36) des Verstärkers (30) vorzusehen, der nötig ist, damit der Verstärker (30) die Hochdruckkammer (86) der Medizineinheit (16) unter Druck setzt,
wobei der Kolben (76) der Medizineinheit (16) und der Kolben (36) des Verstärkers (30) konfiguriert sind, um sich als eine Einheit zu bewegen, so dass, wenn Druck auf den Kolben (36) des Verstärkers (30) von dem Luftverteilungssystem (32) aufgebracht wird, der Kolben (76) der Medizineinheit (16) in der Hochdruckkammer (86) zurückgezogen wird, was bewirkt, dass die Medizin aus der Vorratsflasche (18) in die Hochdruckkammer (86) gezogen wird und zur Einspritzung unter Druck gesetzt wird, und dass auf das Öffnen der Nadelanordnung (118) hin der Kolben (76) der Medizineinheit (16) und der Kolben (36) des Verstärkers (30) freigegeben werden und die unter Druck stehende Medizin in der Hochdruckkammer (86) in den Hochdruckschlauch (68) zum Handstück (20) drücken; und
eine Energieeinheit (12) mit einem Gaszylinder (22), wobei die Energieeinheit (12) Gas in das Luftverteilungssystem (32) der Antriebseinheit (14) liefert.

2. Pistole (10) nach Anspruch 1, wobei die Kugelverschlussanordnung (120) weiter einen ersten Kugelverschluss (130), eine Hauptfeder (134) und einen zweiten Kugelverschluss (132) aufweist, wobei der erste Kugelverschluss (130) das Freigeben der Hauptfeder (134) steuert, und wobei der zweite Kugelverschluss (132) die Reaktivierung der Pistole (10) steuert.

3. Pistole (10) nach einem der Ansprüche 1 oder 2, wobei das Handstück (20) weiter eine Sicherung (136) in Verbindung mit dem Knopf (116) aufweist, wobei, wenn die Sicherung (136) in Eingriff ist, der Knopf (116) die Kugelverschlussanordnung (120) nicht freigeben kann.

4. Pistole (10) nach einem der Ansprüche 1 bis 3, wobei, wenn der Kern (90) gegen das Lebewesen gedrückt wird, der Kern (90) in Kontakt mit dem Knopf (116) kommt.

5. Pistole (10) nach Anspruch 4, wobei der Kern (90) konfiguriert ist, um den Knopf (116) zu drücken, um die Ventilanordnung (110) zu öffnen.

6. Pistole (10) nach Anspruch 4, wobei der Kern (90) konfiguriert ist, um den Knopf (116) zu ziehen, um die Ventilanordnung (110) zu öffnen.

7. Bausatz zum transdermalen Verabreichen von Medizin an ein Lebewesen, welcher Folgendes aufweist:
die Strahlinjektionsvorrichtung bzw. Impfpistole (10) nach einem der Ansprüche 1 bis 6; und
eine Weste (150) mit mindestens einer Tasche (160), die konfiguriert ist, um zumindest ein Handstück (20), eine Vorratsflasche (18), die Antriebseinheit (14) oder die Energieeinheit (12) zu halten.

8. Bausatz nach Anspruch 7, wobei die Weste (150) zumindest drei Taschen (160) aufweist, um sowohl die Vorratsflasche (18) als auch die Antriebseinheit (14) als auch die Energieeinheit (12) zu halten.

9. Bausatz nach Anspruch 7 oder Anspruch 8, wobei die Weste (150) weiter zumindest ein Brustband (152) aufweist, welches mit mindestens einem Rückenteil (154) und mindestens einem Hüftteil (156) verbunden ist.

10. Bausatz nach einem der Ansprüche 7 bis 9, wobei die Weste (150) weiter einen Clip (162) aufweist, der konfiguriert ist, um das Handstück zu halten.

## Revendications

1. Injecteur sans aiguille (10) muni d'une poignée séparée (20) pour administrer un médicament à un sujet, comprenant :
la poignée séparée (20) comportant un corps (88) et un coeur (90) situé au moins partiellement dans le corps (88), le coeur (90) comportant une buse (102) sur une extrémité distale, la buse (102) comportant un orifice (104) qui libère un médicament à partir du coeur (90) dans le sujet ;
un module de médicament (16) comportant une soupape d'entrée 82 agencée pour recevoir un médicament, une soupape de sortie agencée pour contrôler le flux de médicament sortant du module de médicament (16) et une chambre à haute pression (86) en communication de fluide avec le coeur (90) de la poignée séparée (20) par l'intermédiaire d'un tuyau à haute pression (68) pour fournir un médicament à haute vitesse au coeur (90) de la poignée (20), le module de médicament (16) comportant en outre un piston (76) situé au niveau d'une extrémité de la chambre à haute pression (86) ;
et dans lequel la poignée (20) comprend en outre un ensemble formant soupape (110) situé dans le coeur (90) pour contrôler la libération du médicament provenant du module de médicament (16) vers la poignée (20), l'ensemble formant soupape (110) comprenant un ensemble de verrouillage à bille (120) et un ensemble formant aiguille (118),
dans lequel l'ensemble de verrouillage à bille (120) comprend un bouton (116), dans lequel l'activation du bouton (116) provoque la libération de l'ensemble de verrouillage à bille (120), dans lequel lorsque le coeur (90) est pressé contre le sujet, le coeur (90) contacte le bouton (116) et provoque la libération de l'ensemble de verrouillage à bille (120),
dans lequel la libération de l'ensemble de verrouillage à bille (120) provoque un mouvement de l'ensemble formant aiguille (118), dans lequel le mouvement de l'ensemble formant aiguille (118) contrôle la libération du médicament à partir du module de médicament (16) vers la poignée (20) et vers la sortie du coeur (90) de la poignée (20),
une bouteille d'alimentation (18) en communication avec le module de médicament (16), la bouteille d'alimentation (18) fournissant le médicament au module de médicament (16) ;
un module de fourniture d'énergie (14) connecté au module de médicament (16) pour créer une haute pression dans la chambre à haute pression (86), le module de fourniture d'énergie (14) comportant un amplificateur (30) et un système de distribution d'air (32),
dans lequel l'amplificateur (30) convertit de l'énergie pneumatique en pression hydraulique pour pressuriser la chambre à haute pression (86) du module de médicament (16) et dans lequel l'amplificateur (30) comprend un piston (36),
dans lequel le système de distribution d'air fournit et contrôle le débit d'air dans l'amplificateur (30) par l'intermédiaire d'une soupape (42) pour appliquer une pression contre le piston (36) de l'amplificateur (30) nécessaire pour que l'amplificateur (30) pressurise la chambre à haute pression (86) du module de médicament (16),
dans lequel le piston (76) du module de médicament (16) et le piston (36) de l'amplificateur (30) sont agencés pour se déplacer d'un seul bloc, de sorte que lorsqu'une pression est appliquée au piston (36) de l'amplificateur (30) à partir du système de distribution d'air (32), le piston (76) du module de médicament (16) se rétracte dans la chambre à haute pression (86), amenant le médicament provenant de la bouteille d'alimentation (18) à se retirer dans la chambre à haute pression (86) et à être pressurisé pour injection, et de sorte que, à l'ouverture de l'ensemble formant aiguille (118), le piston (76) du module de médicament (16) et le piston (36) de l'amplificateur (30) sont libérés et forcent le médicament pressurisé se trouvant dans la chambre à haute pression (86) à entrer dans le tuyau à haute pression (68) vers la poignée (20) ; et
un module d'énergie (12) comportant un cylindre de gaz (22), le module d'énergie (12) fournissant du gaz au système de distribution d'air (32) du module de fourniture d'énergie (14).

2. Injecteur (10) selon la revendication 1, dans lequel l'ensemble de verrouillage à bille (120) comprend en outre un premier verrouillage à bille (130), un ressort principal (134) et un deuxième verrouillage à bille (132), dans lequel le premier verrouillage à bille (130) contrôle la libération du ressort principal (134) et le deuxième verrouillage à bille (132) contrôle la réactivation de l'injecteur (10).

3. Injecteur (10) selon l'une quelconque des revendications 1 ou 2, dans lequel la poignée (20) comprend en outre une sécurité (136) en communication avec le bouton (116), dans lequel lorsque la sécurité (136) est enclenchée, le bouton (116) ne peut pas libérer l'ensemble de verrouillage à bille (120) .

4. Injecteur (10) selon l'une quelconque des revendications 1 à 3, dans lequel lorsque le coeur (90) est pressé contre le sujet, le coeur (90) contacte le bouton (116).

5. Injecteur (10) selon la revendication 4, dans lequel le coeur (90) est agencé pour pousser le bouton (116) pour ouvrir l'ensemble formant soupape (110).

6. Injecteur (10) selon la revendication 4, dans lequel le coeur (90) est agencé pour tirer le bouton (116) pour ouvrir l'ensemble formant soupape (110).

7. Kit pour administrer de façon transcutanée un médicament à un sujet, comprenant :
l'injecteur sans aiguille (10) de l'une quelconque des revendications 1 à 6 ; et
un gilet (150) comportant au moins une poche (160) agencée pour contenir au moins un élément parmi la poignée (20), une bouteille d'alimentation (18), le module de fourniture d'énergie (14), ou le module d'énergie (12).

8. Kit selon la revendication 7, dans lequel le gilet (150) comporte au moins trois poches (160) pour contenir chacun de la bouteille d'alimentation (18), du module de fourniture d'énergie (14) et du module d'énergie (12).

9. Kit selon la revendication 7 ou la revendication 8, dans lequel le gilet (150) comprend en outre au moins une sangle de poitrine (152) connectée à au moins une partie dorsale (154) et à au moins une partie de ceinture (156).

10. Kit selon l'une quelconque des revendications 7 à 9, dans lequel le gilet (150) comprend en outre une attache (162) agencée pour tenir la poignée.
